# FASCICULE DE BREVET EUROPEEN

(11) **EP 1 080 043 B1**
(45) Date de publication et mention de la délivrance du brevet: **11.09.2002**
(21) Numéro de dépôt: 99915818.1
(22) Date de dépôt: 22.04.1999
(51) Int. Cl.: C02F 3/28, C12M 1/107

(54) **PERFECTIONNEMENTS APPORTES AUX RESERVOIRS ETANCHES POUR LA METHANISATION OU LE STOCKAGE EN AMBIANCE CORROSIVE**
VERBESSERUNGEN AN DICHTEN BEHÄLTERN ZUR METHANISIERUNG ODER FÜR DIE LAGERUNG IN EINER KORROSIVEN UMGEBUNG
IMPROVEMENTS TO SEALED RESERVOIRS FOR METHANATION OR STORAGE IN CORROSIVE ATMOSPHERE

(30) Priorité: 13.05.1998 FR 9806030
(43) Date de publication de la demande: 07.03.2001
(73) Titulaire: ONDEO DEGREMONT, 92500 Rueil-Malmaison (FR)
(72) Inventeur: MALIGE, Jean, Luc, F-92400 Courbevoie (FR); SUHR, Patrick, F-92500 Rueil-Malmaison (FR)
(74) Mandataire: Armengaud Ainé, Alain
(86) Numéro de dépôt international: FR9900962
(87) Numéro de publication internationale: WO99058458

(56) Documents cités:
- GB-A- 2 162 195
- PATENT ABSTRACTS OF JAPAN vol. 005, no. 045 (C-048), 25 mars 1981 (1981-03-25) & JP 55 167091 A (NIIMI TADASHI;OTHERS: 02), 26 décembre 1980 (1980-12-26)

## Description

La présente invention est relative à un dispositif de protection des ouvrages de traitement de boues ou d'eaux résiduaires et elle vise plus particulièrement un dispositif de protection des parties d'ouvrages de méthanisation ou de stockage au contact d'une ambiance corrosive.

Cette invention s'applique particulièrement aux enceintes ou réservoirs étanches dans lesquels sont mis en oeuvre des traitements de fermentation méthanique d'effluents liquides aux concentrations diverses. On sait que la méthanisation, appelée dans certains cas « digestion », est un procédé bactérien traitant la matière organique des effluents suivants : boues issues de stations d'épuration, lisiers de porcs issus d'élevages industriels, eaux résiduaires urbaines ou encore eaux de procédés industriels. Les finalités de la méthanisation sont diverses : production de méthane à des fins énergétiques, stabilisation de la fermentation, réduction de la masse de matière organique, réduction aux normes de la pollution carbonée avant rejet dans l'environnement, etc....

Une enceinte de méthanisation ou de digestion se présente généralement sous la forme d'un réservoir étanche comportant une base en béton sur laquelle repose une virole en acier ou en béton recouverte d'une coupole, ledit réservoir étant rempli d'une liqueur de boue ou de lisier.

Les procédés bactériens mis en oeuvre dans ces enceintes de méthanisation ou de digestion produisent un « biogaz » composé essentiellement de méthane et de gaz carbonique mais qui comprennent également des gaz corrosifs parmi lesquels on peut citer plus particulièrement le sulfure d'hydrogène. Il est donc nécessaire que les matériaux constituant les ouvrages de méthanisation ou de digestion (aciers courants et béton) soient étanches aux gaz corrosifs et qu'ils puissent résister à la corrosion engendrée par ceux-ci.

A l'heure actuelle, la protection des surfaces d'ouvrage en contact avec des gaz corrosifs est réalisée par des revêtements de brai d'époxy, de polyuréthane ou par d'autres peintures équivalentes. Ces revêtements présentent un certain nombre d'inconvénients. Ainsi, dans le cas d'ouvrages en béton :
- nécessité d'appliquer le revêtement sur un béton parfaitement sec, d'où un délai d'attente de 28 jours après achèvement du génie-civil,
- nécessité de préparer toute la surface en vue d'éliminer toute laitance et de reprendre au mortier toute imperfection,
- nécessité, du fait du confinement de l'ouvrage, de mettre en place un système de renouvellement d'air important et travailler avec une peinture sans solvant,
- nécessité de protections vestimentaires strictes pour l'utilisation du brai d'époxy, dont le contact est dangereux. De ce fait, des pays de plus en plus nombreux interdisent cette substance. Le polyuréthane est moins contraignant, mais plus cher,
- risque de déchirure du revêtement et donc ruine de la protection en cas de fissuration de quelques dixièmes de millimètres du béton,
- risque de décollement du revêtement par infiltration d'humidité dans le béton à partir des surfaces non protégées,

Dans le cas d'ouvrages en acier, les contraintes de renouvellement d'air et de protection de l'applicateur sont identiques. S'ajoutent encore, pour ces revêtements de peinture, la nécessité de grenailler la surface avant l'application.

La protection d'un ouvrage acier peut également être obtenue par l'utilisation de tôles en acier vitrifié. Outre son coût plus élevé, cette technique demande des précautions importantes tout au long du chantier pour éviter tout éclat dans la couche de verre. De plus, la différence de dilatation existant entre l'acier et le verre induit un risque de fissuration pour les bâtiments chauffés, particulièrement préoccupant pour des ouvrages de méthanisation à 35°C. Enfin, les joints mastics réalisant l'étanchéité à la jonction des plaques résistent mal à la corrosion des gaz.

Il apparaît donc nettement que, dans l'état de l'art, la réalisation d'un dispositif de protection des ouvrages de méthanisation est malaisée et comporte des aléas préjudiciables à la pérennité des ouvrages.

Par ailleurs, il existe des gazomètres souples pour le stockage du biogaz issu des procédés de méthanisation. Ces gazomètres sont fixés sur un radier en béton et ils sont constitués d'une double membrane : une membrane intérieure réalisant une enceinte étanche destinée à recevoir le biogaz et une membrane extérieure, enveloppant la membrane intérieure et qui, mise en surpression, applique une pression donnée sur le volume de biogaz contenu dans la membrane intérieure. Ces gazomètres souples peuvent également être mis en place directement sur un digesteur classique du type spécifié ci-dessus, à la place de la coupole.

GB-A-2 162 195 décrit une fixation de membrane pour réacteur de fermentation, du type gazomètre, mettant en oeuvre un bourrelet gonflable inséré dans un profilé métallique. Cette publication antérieure ne prévoit pas de moyens permettant d'assurer une étanchéité entre le profilé métallique et la paroi du réacteur et dans ces conditions, le problème découlant de la corrosion due au gaz corrosif n'est pas résolu.

Partant de cet état de la technique, la présente invention se propose de réaliser un méthaniseur/digesteur recouvert d'une coupole de type gazomètre souple dans dans lequel la protection de la virole du méthaniseur/digesteur est réalisée à l'aide de moyens économiques et fiables dans le temps.

La présente invention se propose d'apporter une solution aux problèmes des dispositifs antérieurs rappelés ci-dessus, en se fixant pour objectifs d'éviter le recours à un revêtement anti-corrosion sur les parties de l'ouvrage exposées à l'ambiance corrosive et également de supprimer la nécessité de réaliser une étanchéité entre la partie supérieure de la virole et les moyens prévus pour recouvrir cette dernière, notamment une double membrane étanche.

En conséquence, cette invention a pour objet un réservoir étanche destiné à mettre en oeuvre un traitement de fermentation méthanique d'effluents liquides tels que notamment des boues, lisiers, effluents urbains ou industriels, ou à stocker un milieu en ambiance corrosive, comportant un radier en béton, une virole en acier ou en béton et une coupole étanche et résistante à la corrosion, constituée d'une double membrane : une membrane intérieure délimitant, avec la surface de l'effluent liquide contenu dans ladite virole, une enceinte étanche destinée à recevoir le biogaz résultant de la fermentation méthanique, ou ladite ambiance corrosive et, une membrane extérieure enveloppant la membrane intérieure et qui, mise en surpression, applique une pression donnée sur le volume de biogaz ou de l'ambiance corrosive, contenu dans ladite enceinte, ledit réservoir étanché étant caractérisé en ce que ladite membrane intérieure se prolonge vers le bas par une jupe dont la partie inférieure est immergée sous le niveau de l'effluent liquide contenu dans le réservoir, permettant ainsi d'assurer l'étanchéité de l'enceinte délimitée au-dessus dudit niveau, ladite jupe étant fixée, sur toute sa périphérie, sous le niveau de l'effluent liquide, sur la paroi interne de ladite virole, par l'intermédiaire d'un joint plaqué contre ladite paroi à l'aide de fers plats en acier inoxydable qui sont fixés dans la paroi, cette fixation pouvant être réalisée par exemple à l'aide de boulons, munis de rondelles étanches connues sous la dénomination de « bagues BS ».

Selon l'invention, ledit joint est réalisé de préférence en matériau synthétique, tel que notamment du néoprène

D'autres caractéristiques et avantages de l'invention ressortiront de la description faite ci-après en référence au dessin annexé qui en illustre un exemple de réalisation dépourvu de tout caractère limitatif.

Sur le dessin :
- la figure 1 est une vue schématique en coupe verticale représentant un digesteur selon la présente invention et,
- les figures 2 et 3 sont des vues de détail, également en coupe verticale, à plus grande échelle, illustrant deux exemples de réalisation des moyens prévus par l'invention pour assurer le positionnement et la fixation de la jupe de la membrane interne sur la virole du réservoir.

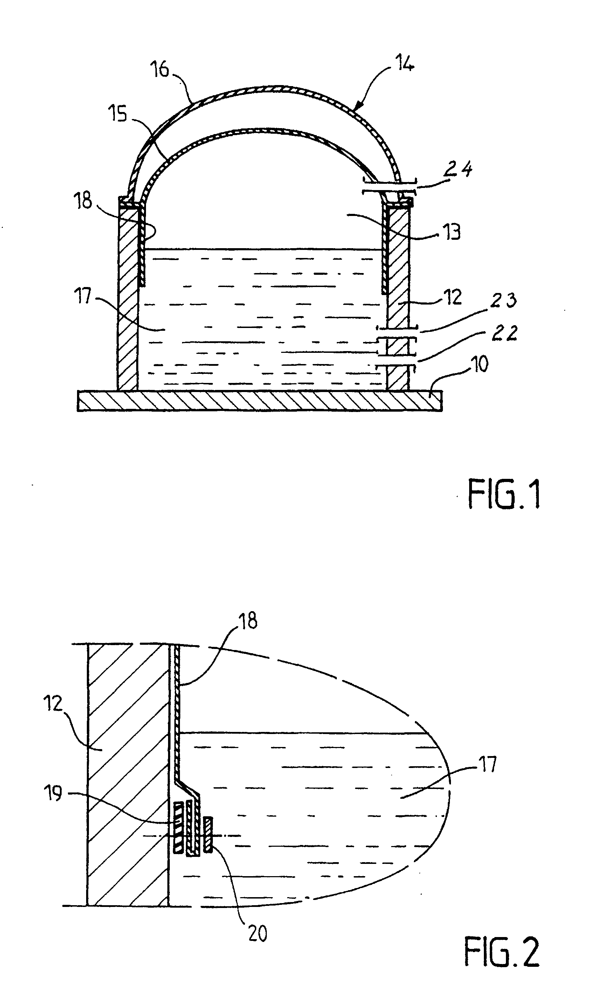

En se référant à la figure 1, celle-ci représente un ouvrage pour la fermentation méthanique mettant en oeuvre la présente invention. Cet ouvrage comporte de façon connue un radier 10 en béton et une virole 12 en acier ou en béton qui est recouverte d'une membrane double réalisée en un matériau étanche et résistant à la corrosion, désignée dans son ensemble par la référence 14. Cette membrane double est composée d'une membrane interne 15 qui délimite, avec la virole 12, une enceinte 13, étanche au biogaz enfermé au-dessus de l'effluent liquide 17 contenu dans le volume défini par la virole et, une membrane externe 16 enveloppant la membrane interne 15, ladite membrane 16 étant différente de la membrane interne 15. Comme dans les solutions antérieures mettant en oeuvre une telle double membrane, la membrane extérieure est mise en surpression. L'alimentation ou l'extraction de boues ou d'effluent est réalisée par l'intermédiaire des passages 22 et 23 scellés dans la virole. L'extraction du biogaz est quant à elle réalisée au niveau d'un passage étanche 24 enserrant les membranes 15 et 16.

Selon la présente invention, la membrane inférieure 15 est prolongée par une jupe circulaire 18 également étanche et résistant à la corrosion, ladite jupe 18 venant recouvrir la surface interne de l'ouvrage depuis le haut de la virole 12 jusqu'à une distance déterminée, en dessous du niveau liquide minimal de l'effluent 17 de manière à assurer une étanchéité totale entre la virole 12 et le « ciel gazeux » corrosif contenu dans l'enceinte 13. A titre d'exemple non limitatif, on mentionnera que la distance séparant la partie inférieure de la jupe 18 du niveau liquide minimal peut être de l'ordre de 1 mètre.

Selon la présente invention, la membrane peut être maintenue en position par la mise en place d'un joint entre la partie inférieure de la jupe 18 et la virole 12. La figure 2 illustre un exemple de réalisation non limitatif d'un tel joint. Celui-ci est constitué ici d'un anneau circulaire de préférence en matériau synthétique, notamment en néoprène, 19 qui est maintenu plaqué la long de la paroi interne de la virole 12 à l'aide de fers plats en acier inoxydable tels que 20, boulonnés dans ladite paroi, en ayant recours à des rondelles étanches du type « bagues BS » par exemple. On peut bien entendu utiliser d'autres moyens permettant d'assurer le maintien en position de la membrane, de manière que la partie inférieure de la jupe soit toujours située en-dessous du niveau de l'effluent liquide 17 contenu dans la virole.

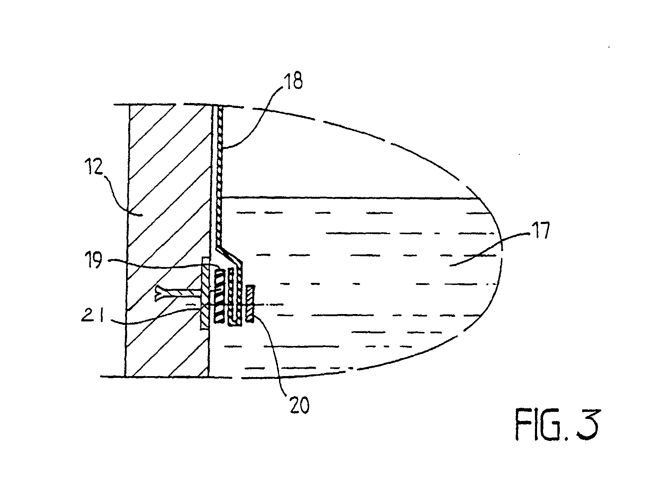

Dans la variante illustrée par la figure 3, la jupe 18 est en outre fixée sur la paroi interne de la virole 12 à l'aide d'une couronne métallique 21 qui est insérée dans la virole et sur laquelle on fixe la jupe

Il résulte de la lecture de la description faite ci-dessus que l'invention permet effectivement de résoudre les problèmes que pose la présence de gaz corrosifs dans les ouvrages de méthanisation ou dans des ouvrages de stockage, tout en éliminant la nécessité de prévoir un revêtement protecteur sur les parties d'ouvrages au contact des gaz corrosifs et en supprimant les problèmes d'étanchéité devant être réalisés en partie supérieure de la virole, l'étanchéité, dans le dispositif selon l'invention étant assuré par la présence de la jupe qui se prolonge en dessous du niveau liquide, l'invention prévoyant un maintien en position et une étanchéité de la membrane inférieure, par tout moyen approprié.

Il en résulte les avantages suivants, dans les phases de réalisation et de fonctionnement des ouvrages ;
- la pose sur la paroi de l'ouvrage peut être réalisée quelque soit l'état de surface de l'acier, l'état de surface ou l'hygrométrie du béton,
- la manipulation et le contact de la jupe sont sans risque, il n'y a pas de renouvellement d'air particulier à réaliser ;
- la fissuration éventuelle du béton n'a pas dé conséquence sur la capacité de protection de la jupe, totalement indépendante.
- l'élasticité du textile permet d'absorber tout phénomène de dilatation différentielle.

Il demeure bien entendu que la présente invention n'est pas limitée à l' exemple de réalisation et d'application décrit et/ou représenté mais qu'elle en englobe toutes les variantes qui entrent dans le cadre de la portée des revendications annexées.

## Revendications

1. Réservoir étanche destiné à mettre en oeuvre un traitement de fermentation méthanique d'effluents liquides tels que notamment des boues, lisiers, effluents urbains ou industriels, ou à stocker un milieu en ambiance corrosive, comportant un radier en béton (10), une virole (12) en acier ou en béton et une coupole (14) étanche et résistante à la corrosion, constituée d'une double membrane : une membrane intérieure (15) délimitant, avec la surface de l'effluent liquide contenu dans ladite virole, une enceinte étanche (13) destinée à recevoir le biogaz résultant de la fermentation méthanique, ou ladite ambiance corrosive et, une membrane extérieure (16) enveloppant la membrane intérieure et qui, mise en surpression, applique une pression donnée sur le volume de biogaz ou de l'ambiance corrosive, contenu dans ladite enceinte (13), ledit réservoir étanche étant **caractérisé en ce que** ladite membrane intérieure (15) se prolonge vers le bas par une jupe (18), dont la partie inférieure est immergée sous le niveau de l'effluent liquide contenu dans le réservoir afin d'assurer l'étanchéité de l'enceinte (13) délimitée au-dessus dudit niveau, ladite jupe étant fixée, sur toute sa périphérie, sous le niveau de l'effluent liquide (17), sur la paroi interne de ladite virole (12), par l'intermédiaire d'un joint (19) plaqué contre ladite paroi, à l'aide de fers plats (20) en acier inoxydable qui sont fixés dans la paroi.

2. Réservoir selon la revendication 1, **caractérisé en ce que** la fixation dans la paroi interne de la virole (12) des fers plats (20) est assurée à l'aide de boulons munis de rondelles étanches.

3. Réservoir selon la revendication 1, **caractérisé en ce qu'**il comporte en outre une couronne métallique (21) qui est insérée dans ladite virole (12) et sur laquelle est fixée la jupe (18).

## Claims

1. Sealed reservoir for performing a methane fermentation treatment on liquid effluents such as in particular sludge, manure, urban or industrial waste or for the storage of a medium in a corrosive atmosphere, which comprises a concrete apron (10), a pressure envelope (12) made from concrete or steel and a tight, corrosion-resistant dome (14) constituted by a double membrane, namely an inner membrane (15) defining, with the surface of the liquid effluent contained in said pressure envelope, a tight chamber (13) for receiving the biogas resulting from the methane fermentation, or said corrosive atmosphere and an outer membrane (16) enveloping the inner membrane and which, placed under an excess pressure, applies a given pressure to the biogas volume or to the corrosive atmosphere contained in said chamber (13), said sealed reservoir being **characterized in that** said inner membrane (15) is extended downwards by a skirt (18), whereof the lower part is immersed beneath the level of the liquid effluent contained in the reservoir in order to ensure the tightness of the chamber (13) defined above said level, said skirt being fixed over its entire periphery beneath the level of the liquid effluent (17), to the inner wail of the pressure envelope (12), by means of a joint (19) engaged against said wall, with the aid of stainless steel, flat irons (20), which are fixed in the wall.

2. Reservoir according to claim 1, **characterized in that** fixing in the inner wall of the pressure envelope (12) of the flat irons (20) takes place with the aid of bolts provided with tight washers.

3. Reservoir according to claim 1, **characterized in that** it also has a metal ring (21) inserted in said pressure envelope (12) and to which is fixed the skirt (18).

## Patentansprüche

1. Behälter, der dicht ist und in welchem eine Methangärungsbehandlung von flüssigen Abprodukten wie insbesondere Schlamm, Gülle und kommunalem bzw. industriellem Abwasser durchgeführt oder ein Medium in einer korrosiven Atmosphäre gespeichert werden soll, welcher eine Betonfundamentplatte (10), einen Stahl- oder Betonmantel (12) und eine dichte und korrosionsbeständige gewölbte Decke (14) umfasst, die aus einer Doppelhülle, einer Innenhülle (15), die mit der Oberfläche des im Behälter enthaltenen flüssigen Abprodukts einen dichten Raum (13) begrenzt, der vorgesehen ist, das bei der Methangärung entstehende Biogas oder die korrosive Atmosphäre aufzunehmen, und aus einer Außenhülle (16) besteht, welche die Innenhülle umgibt und, unter Überdruck gesetzt, einen vorgegebenen Druck auf das Volumen aus Biogas oder korrosiver Atmosphäre ausübt, das in dem Raum (13) enthalten ist, **dadurch gekennzeichnet, dass** sich die Innenhülle (15) durch eine Schürze (18) nach unten verlängert, deren unterer Teil unter den Spiegel des im Behälter enthaltenen flüssigen Abprodukts eintaucht, um die Dichtigkeit des Raums (13), der über diesem Spiegel gebildet wird, sicherzustellen, wobei die Schürze über den gesamten Umfang unter dem Spiegel des flüssigen Abprodukts (17) an der Innenwand des Mantels (12) über eine Dichtverbindung (19) befestigt ist, die an diese Wand mittels in der Wand befestigter Flacheisen (20) aus rostfreiem Stahl gepresst wird.

2. Behälter nach Anspruch 1, **dadurch gekennzeichnet, dass** die Flacheisen (20) durch mit dichten Unterlegscheiben versehene Schraubenbolzen an der Innenwand des Mantels (12) befestigt sind.

3. Behälter nach Anspruch 1, **dadurch gekennzeichnet, dass** er außerdem einen Metallring (21) enthält, der in den Mantel (12) eingefügt ist und an welchem die Schürze (18) befestigt ist.
